# FASCICULE DE BREVET EUROPEEN

(11) **EP 3 377 061 B1**
(45) Date de publication et mention de la délivrance du brevet: **30.12.2020**
(21) Numéro de dépôt: 16812992.2
(22) Date de dépôt: 17.11.2016
(51) Int. Cl.: A61K 31/426, A61K 9/00, A61P 27/02

(54) **MIRABÉGRON POUR LE TRAITEMENT DE MALADIES RÉTINIENNES**
MIRABEGRON ZUR BEHANDLUNG VON NETZHAUTERKRANKUNGEN
MIRABEGRON FOR THE TREATMENT OF RETINAL DISEASES

(30) Priorité: 17.11.2015 FR 1561067
(43) Date de publication de la demande: 26.09.2018
(73) Titulaire: Sorbonne Université, 75006 Paris (FR); Centre National de la Recherche Scientifique, 75016 Paris (FR); INSERM (Institut National de la Santé et de la Recherche Médicale), 75654 Paris Cedex 13 (FR)
(72) Inventeur: FONTAINE, Valérie, 94230 Cachan (FR); VIDAL, Cécile, 94340 Joinville-le-Pont (FR); SAHEL, José-Alain, 75013 Paris (FR)
(74) Mandataire: Icosa
(86) Numéro de dépôt international: PCT/FR2016/052981
(87) Numéro de publication internationale: WO 2017/085407

(56) Documents cités:
- EP-A1- 1 028 111
- WO-A2-2008/042399
- WO-A2-2010/147830
- JENA J. STEINLE ET AL: "[beta] 3 -Adrenergic Receptors Regulate Retinal Endothelial Cell Migration and Proliferation", JOURNAL OF BIOLOGICAL CHEMISTRY, vol. 278, no. 23, 6 juin 2003 (2003-06-06) , pages 20681-20686, XP055274900, US ISSN: 0021-9258, DOI: 10.1074/jbc.M300368200
- STEINLE J J ET AL: "beta3-Adrenergic receptors mediate choroidal endothelial cell invasion, proliferation, and cell elongation", EXPERIMENTAL EYE RESEARCH, ACADEMIC PRESS LTD, LONDON, vol. 80, no. 1, 1 janvier 2005 (2005-01-01), pages 83-91, XP004712453, ISSN: 0014-4835, DOI: 10.1016/J.EXER.2004.08.015

## Description

### DOMAINE DE L'INVENTION

La présente demande concerne le traitement de maladies rétiniennes, telle que la dégénérescence maculaire liée à l'âge. La présente demande concerne en particulier l'utilisation du mirabégron ou d'un de ses analogues, sels ou solvates pour le traitement d'une maladie rétinienne, en particulier pour le traitement de la dégénérescence maculaire liée à l'âge.

### ÉTAT DE LA TECHNIQUE

La dégénérescence maculaire liée à l'âge (DMLA) est une cause majeure de cécité (au sens légal) dans les pays développés et le trouble oculaire le plus courant chez les personnes âgées. La DMLA est caractérisée par une dégénérescence du neuroépithélium dans la zone maculaire de l'œil. Deux formes majeures de DMLA de stade avancé peuvent être distinguées : la DMLA néovasculaire et la DMLA atrophique.

La DMLA néovasculaire, dite humide ou exsudative, se traduit par une prolifération de nouveaux vaisseaux anormaux sous la rétine. Ce phénomène est appelé « néovascularisation choroïdienne » ou « NVC ». Ces nouveaux vaisseaux fragiles laissent diffuser du sérum, responsable d'un soulèvement de la rétine, et/ou du sang, entraînant l'apparition d'hémorragies rétiniennes. La DMLA néovasculaire est la cause majeure de cécité des personnes âgées dans les pays industrialisés. Plusieurs traitements ont été développés pour améliorer la situation clinique des patients, notamment par des thérapies ciblant VEGFA, un puissant stimulateur de l'angiogenèse et de la perméabilité vasculaire.

La DMLA atrophique, aussi connue sous le nom d'atrophie géographique ou de DMLA sèche, correspond à la disparition progressive des cellules de l'épithélium pigmentaire rétinien (EPR), puis à celle des photorécepteurs situés au niveau de la macula. Ce processus génère des trous de taille croissante dans la macula, visibles par une simple observation de la rétine (fond d'œil).

L'incidence de la DMLA néovasculaire et de la DMLA atrophique est comparable mais l'expansion des lésions atrophiques et des troubles visuels associés sont généralement plus lents dans le cas de la DMLA atrophique. Il s'écoule en général entre cinq et dix ans avant que le patient ne perde sa vision centrale. Actuellement, il n'existe pas de thérapie approuvée pour prévenir ou traiter la DMLA atrophique, principalement en raison de l'absence d'identification de molécules cibles. Certaines études ont démontré que la consommation de vitamines E et C, de béta caroténoïdes et de zinc pourrait ralentir le développement de la DMLA atrophique. Cependant, la progression de la maladie n'est pas stoppée.

Des études ont montré que l'accumulation de lipofuscine, un pigment cellulaire composé de débris de molécules, dans les cellules de l'EPR est un marqueur associé à la forme atrophique de la DMLA (Nandakumar et al, Seminars in ophthalmology. 2012, 27(5-6):197-201; Schmitz-Valckenberg et al, Survey of ophthalmology. 2009,54(1): 96-117). Un défaut dans la digestion des segments externes des photorécepteurs par l'EPR est à l'origine de cette accumulation et est probablement lié à une baisse d'activité des enzymes lysosomales (Mahon et al, Curr Eye Res. 2004, 28:277-284). En effet, l'activité des enzymes lysosomales est maximale dans une gamme de pH très acide. Une augmentation du pH lysosomal des cellules de l'EPR réduit ainsi ce processus digestif indispensable au bon fonctionnement rétinien.

La demande de brevet internationale WO 2008/042399 décrit une méthode de traitement de la DMLA par restauration d'un pH lysosomal acide. Cette demande de brevet décrit également que la stimulation de récepteurs adénosine ou beta-adrénergiques pourrait diminuer le pH lysosomal.

Cependant, la Demanderesse a montré que certaines molécules connues pour activer les récepteurs béta-adrénergiques, bien que diminuant le pH lysosomal des cellules de l'EPR, n'induisent pas la digestion des segments externes des photorécepteurs (voir les Exemples), et ne permettent donc pas de traiter la DMLA.

De plus, aucune molécule n'a encore été validée cliniquement à l'heure actuelle.

Ainsi, il existe toujours un besoin d'identifier les molécules assurant une activité lysosomale optimale afin de permettre la dégradation des segments externes des photorécepteurs dans les cellules de l'EPR, permettant ainsi de prévenir et/ou de traiter la DMLA.

La Demanderesse a démontré, de façon surprenante, que certains agonistes aux récepteurs adrénergiques, tels que le mirabégron, réduisent significativement l'accumulation de lipofuscine dans les cellules de l'EPR.

La présente invention concerne donc l'utilisation du mirabégron, ou d'un sel ou solvate de celui-ci pour le traitement d'une maladie rétinienne, telle que la dégénérescence maculaire liée à l'âge.

### RÉSUMÉ

La présente invention concerne un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation dans le traitement d'une maladie rétinienne affectant la macula chez un sujet.

Dans un mode de réalisation, ledit anilide de l'acide (R)-2-(2-aminothiazol-4-yl)-4'-[2-[(2-hydroxy-2-phényléthyl)amino]éthyl]acétique est le mirabégron, un sel ou un solvate pharmaceutiquement acceptable de celui-ci.

Dans un mode particulier de réalisation, ladite maladie rétinienne est la dégénérescence maculaire liée à l'âge, de préférence la dégénérescence maculaire liée à l'âge de type atrophique.

La présente invention concerne également une composition pharmaceutique comprenant un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation telle que décrite ci-dessus et au moins un véhicule pharmaceutiquement acceptable.

La présente invention concerne en outre un médicament comprenant un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation telle que décrite ci-dessus.

Dans un mode de réalisation, ladite composition pharmaceutique ou ledit médicament selon l'invention est destiné à être administré au sujet qui en a besoin par voie orale ou par voie topique.

La présente invention concerne également un kit, comprenant un composé, une composition pharmaceutique, ou un médicament tels que décrits ci-dessus.

Dans un mode de réalisation, ledit kit est caractérisé en ce qu'il comprend en outre un appareil servant à administrer ledit composé, ladite composition pharmaceutique ou ledit médicament à un sujet qui en a besoin, et optionnellement les instructions pour l'administration dudit composé, de ladite composition pharmaceutique ou dudit médicament audit sujet.

En outre, la présente invention concerne le mirabégron pour son utilisation pour le traitement de la DMLA.

### DÉFINITIONS

Dans la présente invention, les termes ci-dessous sont définis de la manière suivante :
- Un « **sel pharmaceutiquement acceptable** » du composé de l'invention inclut les sels d'addition à un acide ou à une base dudit composé. Des sels d'addition à un acide appropriés sont formés à partir d'acides qui forment des sels non toxiques. Des exemples de sels d'addition à un acide incluent, mais ne sont pas limités aux sels d'acétate, de trifluoroacétate, d'adipate, d'aspartate, de benzoate, de besylate, de bicarbonate/carbonate, de bisulphate/sulphate, de borate, le tetrafluoroborate, de camsylate, de citrate, de cyclamate, d'édisylate, d'esylate, de formate, de fumarate, de gluceptate, de gluconate, de glucuronate, d'hexafluorophosphate, d'hibenzate, d'hydrochloride/chloride, d'hydrobromide/bromide, d'hydroiodide/iodide, d'isethionate, de lactate, de malate, de maléate, de malonate, de mésylate, de méthylsulphate, de naphthylate, de 2-napsylate, de nicotinate, de nitrate, d'orotate, d'oxalate, de palmitate, de pamoate, de phosphate/hydrogen phosphate/dihydrogen phosphate, de pyroglutamate, de saccharate, de stéarate, de succinate, de tannate, de tartrate, de tosylate, de trifluoroacetate et de xinofoate. Des sels d'addition à une base appropriés sont formés à partir de bases qui forment des sels non toxiques. Des exemples de sels d'addition à une base incluent, mais ne sont pas limités aux sels d'aluminium, d'arginine, de benzathine, de calcium, de choline, de diéthylamine, de diolamine, de glycine, de lysine, de magnésium, de méglumine, d'olamine, de potassium, de sodium, de trométhamine, de 2-(diéthylamino)éthanol, d'éthanolamine, de morpholine, 4- (2- hydroxyéthyl)morpholine et de zinc. De préférence, les sels pharmaceutiquement acceptables incluent l'hydrochloride/chloride, l'hydrobromide/bromide, le bisulphate/sulphate, le nitrate, le citrate, et l'acétate.
- Le terme « **solvate** » est utilisé dans la présente invention pour décrire un composé de l'invention comprenant des quantités stoechiométriques ou sous-stoechiométriques d'une ou de plus d'une molécule solvant pharmaceutiquement acceptable telle que l'éthanol.
- Le terme « **sujet** » concerne un mammifère, de préférence un humain. Dans un mode de réalisation, le sujet peut être un « patient », i.e. un animal à sang chaud, de préférence un humain, en attente de recevoir ou recevant des soins médicaux, qui a fait l'objet d'une procédure médicale, ou qui est suivi pour le développement d'une maladie rétinienne. Dans un mode de réalisation, le sujet est un adulte, par exemple un sujet ayant plus de 18 ans. Dans un autre mode de réalisation, le sujet est un enfant, par exemple un sujet ayant moins de 18 ans. Dans un mode de réalisation, le sujet est un homme. Dans un autre mode de réalisation, le sujet est une femme.
- Les termes « **traitement** » ou « **traiter** » concernent à la fois le traitement thérapeutique et les mesures prophylactiques ou préventives, dont l'objet est d'empêcher ou de ralentir la progression d'une maladie rétinienne. Les sujets qui ont besoin d'un traitement incluent ceux qui ont déjà une maladie rétinienne, ceux prédisposés à une maladie rétinienne et ceux chez qui une maladie rétinienne doit être prévenue. Un sujet est traité avec succès pour une maladie rétinienne si, après avoir reçu une quantité thérapeutiquement efficace d'un composé de l'invention, le patient montre une réduction observable ou mesurable, ou l'absence, de l'un au moins des points suivants : réduction du nombre de cellules pathogéniques, réduction du pourcentage de cellules pathogéniques par rapport aux cellules totales, et/ou de l'un ou de plusieurs des symptômes associés à la maladie rétinienne, une amélioration de l'acuité visuelle ou une amélioration de la qualité de vie. Les paramètres d'évaluation ci-dessus sont facilement mesurables par les procédures de routine familières à un médecin.
- Le terme « **véhicule** » concerne une substance qui porte le produit d'intérêt dans une composition, en particulier ce peut être une substance qui permet de le dissoudre. Le véhicule peut par exemple être de l'eau.
- Un « **véhicule pharmaceutiquement acceptable** » concerne un véhicule qui ne produit pas de réaction défavorable, allergique ou indésirable lorsqu'il est administré à un sujet. Cela inclut tous les solvants, les milieux de dispersion, les enrobages, les agents antibactériens et antifongiques, les agents isotoniques, les agents à absorption retardée et autres substances similaires. Pour une administration chez un être humain, les préparations doivent répondre aux critères de stérilité, de pyrogénicité, et aux normes de sécurité et de pureté générales requises par les offices régulateurs tels que la FDA ou l'EMA.
- Une « **quantité thérapeutiquement efficace** » concerne la quantité d'agent thérapeutique nécessaire et suffisante, sans causer d'effets négatifs ou indésirables significatifs, pour (1) retarder ou empêcher l'apparition de la maladie rétinienne ; (2) diminuer ou stopper la progression, l'aggravation ou la détérioration d'un ou de plusieurs des symptômes de la maladie rétinienne ; (3) soulager ou apporter des améliorations aux symptômes de la maladie rétinienne ; (4) réduire la sévérité ou l'incidence de la maladie rétinienne, et/ou (5) guérir la maladie rétinienne. Une quantité thérapeutiquement efficace peut être administrée avant l'apparition de la maladie rétinienne, pour une action préventive ou prophylactique. De manière alternative ou additionnelle, la quantité thérapeutiquement efficace peut être administrée après l'initiation de la maladie rétinienne, pour une action thérapeutique.
- « **Environ** », placé devant un nombre, signifie plus ou moins 10% de la valeur nominale de ce nombre.

### DESCRIPTION DÉTAILLÉE

La présente demande concerne l'utilisation d'un composé pour le traitement d'une maladie rétinienne chez un sujet qui en a besoin, ledit composé étant un agoniste des récepteurs adrénergiques.

De préférence, dans un mode de réalisation, le composé de l'invention est un agoniste des récepteurs adrénergiques béta 1, 2 ou 3, de préférence béta 3.

Dans un mode de réalisation, le composé de l'invention est un anilide de l'acide (R)-2-(2-aminothiazol-4-yl)-4'-[2-[(2-hydroxy-2-phényléthyl)amino]éthyl]acétique ou un analogue, un sel ou un solvate pharmaceutiquement acceptable de celui-ci.

Dans le présent texte, l'anilide d'acide (R)-2-(2-aminothiazol-4-yl)-4'-[2-[(2-hydroxy-2-phényléthyl)amino]éthyl]acétique est également référencé sous le terme mirabégron.

Ainsi, le composé de l'invention est le mirabégron.

La Demanderesse a en particulier démontré que le mirabégron diminue le pH lysosomal à faible concentration (1 pM, voir les Exemples et la Figure 1). De plus, la Demanderesse a mis en évidence que le mirabégron restaure de manière significative l'activité de la cathepsine D, une enzyme protéolytique lysosomale requérant un pH acide pour son activité (voir les Exemples et la Figure 2). Ces résultats ont été confirmés dans un modèle cellulaire d'accumulation de lipofuscine. En effet, la Demanderesse a démontré que le mirabégron réduit l'accumulation de lipofuscine dès 2 semaines de traitement (voir les Exemples et la Figure 3). Ainsi, la Demanderesse a démontré le potentiel thérapeutique de cette molécule pour le traitement de la DMLA.

La demande concerne donc le mirabégron ou un analogue, un sel ou un solvate pharmaceutiquement acceptable de celui-ci pour son utilisation dans le traitement d'une maladie rétinienne.

Le mirabégron a la formule générale suivante :

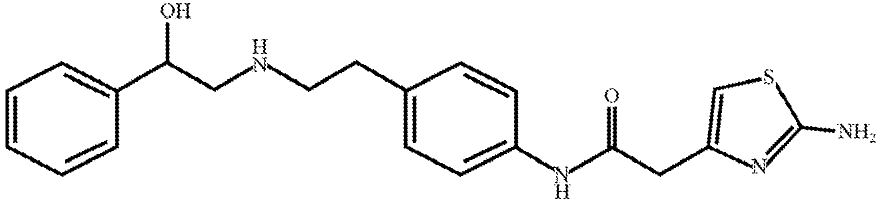

Le mirabégron est également connu sous le nom de Betmiga™, Betanis™ ou Myrabetriq™.

Des exemples d'analogues du mirabégron incluent, sans y être limités, les composés décrits dans le brevet US6346532.

Ainsi, un analogue du mirabégron a la formule générale suivante (I) : 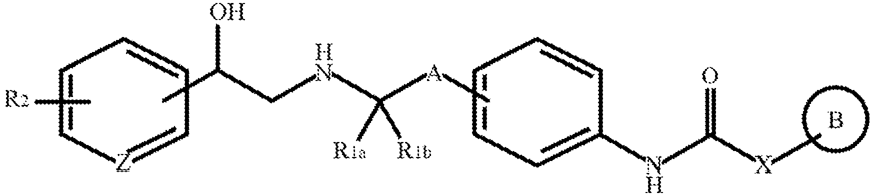 dans laquelle :
- le cycle B représente un groupe hétéroaryle pouvant être substitué et pouvant être fusionné avec un cycle benzénique ;
- X représente une liaison, un alkylène inférieur ou un alcénylène inférieur pouvant être substitué par un groupe hydroxy ou un groupe alkyle inférieur, un carbonyle, ou un groupe représenté par - NH - (lorsque X est un groupe alkylène inférieur pouvant être substitué par un groupe alkyle inférieur, les atomes d'hydrogène liés au carbone constituant le cycle B peuvent former un groupe alkylène inférieur avec le groupe alkyle inférieur, formant ainsi un cycle) ;
- A représente un alkylène inférieur ou un groupe représenté par -alkylène inférieur-O- ;
- R1a et R2a peuvent être identiques ou différents, chacun représentant un atome d'hydrogène ou un groupe alkyle inférieur ;
- R2 représente un atome d'hydrogène ou un atome d'halogène ; et
- Z représente un atome d'azote ou un groupe représenté par =CH-.

Dans le présent texte, le terme « inférieur » signifie une chaine hydrocarbonée linéaire ou ramifiée ayant de 1 à 6 atomes de carbone, sauf spécification contraire.

Des exemples de « groupe alkyle inférieur » incluent, mais ne sont pas limités au méthyle, éthyle, propyle linéaire ou ramifié, butyle linéaire ou ramifié, pentyle linéaire ou ramifié, et hexyle linéaire ou ramifié, de préférence le groupe alkyle inférieur est un alkyle ayant de 1 à 4 atomes de carbone, et particulièrement le méthyle, l'éthyle, le propyle et l'isopropyle.

Un exemple non limitatif de « groupe alkylène inférieur » est un groupe divalent obtenu par soustraction d'un nombre arbitraire d'atome(s) d'hydrogène du « groupe alkyle inférieur » défini ci-dessus, de préférence un groupe alkylène ayant de 1 à 4 atomes de carbone, et particulièrement le méthylène, l'éthylène, le propylène et le butylène.

Des exemples de « groupe alcénylène inférieur » incluent, mais ne sont pas limités aux groupes vinylène, propénylène, buténylène, penténylène et hexénylène.

Dans le présent texte, le « groupe hétéroaryle pouvant être fusionné avec un cycle benzénique » dans le « groupe hétéroaryle pouvant être substitué ou pouvant être fusionné avec un cycle benzénique » signifie un groupe cyclique dans lequel le cycle benzénique est fusionné avec un groupe hétéroaryle tel que décrit ci-après ou un groupe hétéroaryle non fusionné.

Des exemples de « groupe cyclique dans lequel le cycle benzénique est fusionné avec un groupe hétéroaryle » incluent, mais ne sont pas limités au quinolyle, isoquinolyle, quinazolinyle, quinolidinyle, quinoxalinyle, cinnolinyle, benzimidazolyle, l'imidazopyridyle, benzofuranyle, benzoisoxazolyle, benzoxazolyle, benzothiazolyle, oxazolopyridyle, isothiazolopyridyle et benzothiazolyle ; et les cycles additionnés d'oxygènes tels que l'oxobenzofurayle.

Des exemples de « groupe hétéroaryle non fusionné » incluent, mais ne sont pas limités aux groupes hétéroaryles monocycliques tels que le furyle, le thienyle, le pyrrolyle, l'imidazolyle, le thiazolyle, le pyrazolyle, l'isothiazolyle, le pyridyle, le pyrimidyle, le pyridazinyle, le pyrazinyle, le thiadiazolyle, le triazolyle et le tétrazolyle ; et les groupes hétéroaryles bicyliques tels que le naphthylidinyle et le pyridopyrimidinyle.

Le substituant dans le « groupe hétéroaryle pouvant être substitué et pouvant être fusionné avec un cycle benzénique » peut être tout groupe usuellement substitué sur ce groupe cyclique. Des exemples incluent, mais ne sont pas limités à, un atome d'halogène, un alkyle inférieur, un alcényle inférieur, un alcynyle inférieur, un groupement hydroxy, sulfanyle, halogénoalkyle inférieur, alkyle inférieur-O-, alkyle inférieur-S-, alkyle inférieur-O-CO-, carboxy, sulfonyle, sulfinyle, alkyle inférieur-SO-, alkyle inférieur -SO₂-, alkyle inférieur-CO-, alkyle inférieur-CO-O-, carbamoyle, alkyle inférieur -NH-CO-, di-alkyle inférieur-N-CO-, nitro, cyano, amino, guanidino, alkyle inférieur-CO-NH-, alkyle inférieur-SO₂-NH-, alkyle inférieur-NH-, di-alkyle inférieur-N-, O-alkylène inférieur-O- et autres groupements similaires.

Ces substituants peuvent être également substitués par un substituant tel qu'un groupe aryle, un groupe hétéroaryle, un atome d'halogène, un groupement hydroxy, sulfanyle, halogénoalkyle inférieur, alkyle inférieur-O-, alkyle inférieur-S-, alkyle inférieur -O-CO-, carboxy, sulfonyle, sulfinyle, alkyle inférieur-SO-, alkyle inférieur-SO₂-, alkyle inférieur-CO-, alkyle inférieur-CO-O-, carbamoyle, alkyle inférieur-NH-CO-, di-alkyle inférieur-N-CO-, nitro, cyano, amino, guanidino, alkyle inférieur-CO-NH-, alkyle inférieur-SO₂-NH, alkyle inférieur-NH-, di-alkyle inférieur-N- et autres groupements similaires. Ces substituants tels qu'un groupe aryle, un groupe hétéroaryle ou autre peuvent également être substitués par un atome d'halogène, etc...

Le « groupe alcényle inférieur » est un groupe alcényle linéaire ou ramifié ayant de 2 à 6 atomes de carbone. Des exemples incluent, mais ne sont pas limités au vinyle, propényl, butényl, pentényl, et hexényle.

Le « groupe alcynyle inférieur » est un groupe alcynyle linéaire ou ramifié ayant de 2 à 6 atomes de carbone. Des exemples incluent, mais ne sont pas limités à l'éthynyle, le propynyle, le butynyle, le pentynyle et l'hexynyle.

Le terme « atome d'halogène » signifie un atome de fluor, un atome de chlore, un atome de bromure ou un atome d'iode. Le terme « groupe halogénoalkyle inférieur » signifie un groupe dans lequel un (des) atome(s) d'hydrogène du groupe alkyle décrit ci-dessus, choisi(s) arbitrairement, choisis arbitrairement, est (sont) substitué(s) par un (des) atomes d'halogène.

Le cas dans lequel X est une liaison signifie que l'atome de carbone du groupe -CO- est directement lié au cycle B.

Le composé selon la demande, de préférence le mirabégron ou un analogue, comprend au moins un atome de carbone asymétrique. Il existe ainsi des isomères optiques tels que les composés de configuration (R) ou (S), les racémates, les diastéréoisomères, etc... La présente invention inclut l'ensemble des isomères, chacun des isomères isolés et leurs mélanges. La présente invention inclut également les hydrates, les solvates (tels que les solvates d'éthanol) et les substances polymorphiques du composé de l'invention, du mirabégron ou d'un de ses analogues.

Dans un mode de réalisation, l'analogue du mirabégron a la formule générale suivante (la) : 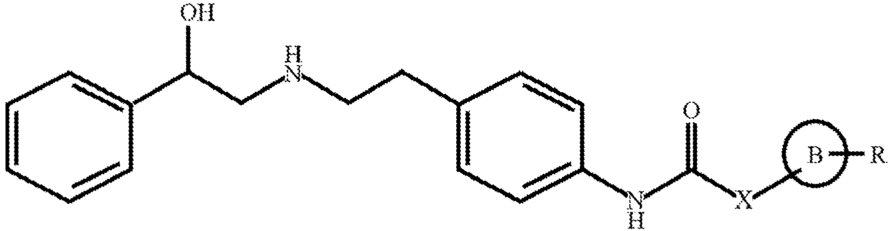 dans laquelle :
- le cycle B représente un groupe hétéroaryle ;
- X représente une liaison ou un groupe alkylène inférieur ;
- R représente un atome d'hydrogène, un atome d'halogène, un groupe alkylène inférieur, un groupe azoté, un groupe alkyle inférieur aryle, ou un groupe halogénoalkyle inférieur aryle ; ou un sel de celui-ci.

Dans un mode de réalisation, l'analogue du mirabégron est sélectionné parmi le groupe comprenant (R)-4'-[2-[(2-Hydroxy-2-phényléthyl)amino]éthyl] -2-pyridinecarboxyanilide, (R)-2-[1-(4-chlorobenzyl)-1H-imidazol-2-yl)-4'-[2-[(2-hydroxy-2-phényléthyl)amino]éthyl]-acétanilide, (R)-2-[1-(3,4-dichlorobenzyl)-1H-tétrazol-5-yl]-4'-[2-[(2-hydroxy-2-phényléthyl)amino]éthyl]acétanilide, (R)-2-(2-aminothiazol-4-yl)-4'-[2-(2-hydroxy-2-phényléthyl)amino]éthyl]acétanilide, (R)-2-(2-benzyl-1H-1,2,4-triazol-3-yl)-4'-[2-[(2-hydroxy-2-phényléthyl)-amino]éthyl]acétanilide, (R)-2-(2-aminopyridin-6-yl)-4'-[2-[(2-hydroxy-2-phényléthyl)amino]éthyl]acétanilide, (R)-4'-[2-[(2-hydroxy-2-phényléthyl)amino]éthyl]-2-(2-pyridyl)acétanilide, (R)-4'-[2-[(2-hydroxy-2-phényléthyl)-amino]éthyl)-2-(2-pyrazinyl)acétanilide, et (R)-4'-[2-[(2-hydroxy-2-phényléthyl)amino]éthyl)-2-(2-pyrimidinyl)-acétanilide, ou l'un de leurs sels.

Dans un mode de réalisation, l'anilide de l'acide (R)-2-(2-aminothiazol-4-yl)-4'-[2-[(2-hydroxy-2-phényléthyl)amino]éthyl]acétique ou un analogue, un sel ou un solvate pharmaceutiquement acceptable de celui-ci n'est pas sous forme lourde ou deutérée. Dans un mode particulier de réalisation, le mirabégron est non deutéré.

Dans un mode de réalisation, le mirabégron est sous forme cristalline. Dans un mode particulier de réalisation, le mirabégron est sous forme cristalline de forme alpha. Dans un autre mode particulier de réalisation, le mirabégron est sous forme cristalline de forme beta. Les formes cristallines alpha et beta du mirabégron sont de base libre et possèdent des caractéristiques physico-chimiques spécifiques. Les formes cristallines alpha et beta du mirabégron sont décrites dans le brevet US7342117.

Dans le présent texte, le terme « maladie rétinienne » englobe les différents troubles pouvant affecter la rétine qui est la couche de cellules nerveuses recouvrant l'arrière de l' œil.

Des exemples de troubles affectant la rétine incluent, mais ne sont pas limités à, la dégénérescence maculaire liée à l'âge (DMLA), la maladie de Stargardt, la rétinopathie diabétique, et la rétinite pigmentaire.

Selon l'invention, la maladie rétinienne est une maladie qui affecte la macula, i.e. la zone centrale de la rétine. Des exemples de maladies affectant la macula incluent, mais ne sont pas limités à la dégénérescence maculaire liée à l'âge et la maladie de Stargardt.

Selon un mode de réalisation, la maladie rétinienne de l'invention est la dégénérescence maculaire liée à l'âge ou la maladie de Stargardt.

Dans un mode de réalisation, la maladie rétinienne de l'invention est la dégénérescence maculaire liée à l'âge.

Dans un mode de réalisation, la dégénérescence maculaire liée à l'âge selon l'invention est au stade précoce, également appelée maculopathie liée à l'âge. La dégénérescence maculaire liée à l'âge de stade précoce est caractérisée par l'accumulation dans et autour de la macula de déchets du fonctionnement des photorécepteurs (appelés « drusen »), associés à des taches pigmentées (altérations de l'épithélium pigmentaire).

Dans un autre mode de réalisation, la dégénérescence maculaire liée à l'âge selon l'invention est au stade tardif. Les stades tardifs se caractérisent par des complications uni ou bilatérales. Deux formes sont alors distinguées, exsudative ou atrophique.

Dans un mode de réalisation, la dégénérescence maculaire liée à l'âge est de type atrophique, également appelée DMLA sèche.

Dans un autre mode de réalisation, la maladie rétinienne de l'invention est la maladie de Stargardt. La maladie de Stargardt est une dystrophie maculaire héréditaire, qui se manifeste chez les enfants entre l'âge de 7 et 12 ans généralement.

Dans un mode de réalisation, le sujet est atteint d'une maladie rétinienne, de préférence la DMLA ou la maladie de Stargardt. Dans un mode de réalisation, le sujet est atteint de DMLA à un stade précoce. Dans un autre mode de réalisation, le sujet est atteint de DMLA à un stade tardif.

Dans un autre mode de réalisation, le sujet est susceptible d'être atteint d'une maladie rétinienne, de préférence de DMLA. Dans un mode de réalisation, le sujet est un sujet à risque pour l'apparition de la maladie rétinienne selon l'invention. Des exemples de risques incluent, mais ne sont pas limités à, l'hérédité (existence présente ou passée d'autres cas de maladie rétinienne, de préférence de DMLA, dans la famille du sujet), le tabagisme, l'âge, l'exposition au soleil, une alimentation déséquilibrée (par exemple faible apport en légumes verts et en acide gras oméga-3), une concentration élevée de cholestérol dans le sang, une pression artérielle élevée, et des facteurs similaires.

Dans un mode de réalisation, le sujet n'a pas encore été traité avec un autre traitement pour la maladie rétinienne selon l'invention. Dans un autre mode de réalisation, le sujet a déjà été traité avec un autre traitement pour la maladie rétinienne selon l'invention. Dans un mode de réalisation, le sujet est un être humain de plus de 45 ans. Dans un autre mode de réalisation, le sujet est un être humain de moins de 18 ans.

La présente invention concerne également une composition comprenant un composé selon l'invention.

Dans un mode de réalisation, la composition de l'invention comprend un anilide de l'acide (R)-2-(2-aminothiazol-4-yl)-4'-[2-[(2-hydroxy-2-phényléthyl)amino]éthyl]acétique ou un sel ou un solvate pharmaceutiquement acceptable de celui-ci, de préférence le mirabégron.

Dans un mode de réalisation, la composition de l'invention est utilisée pour le traitement d'une maladie rétinienne affectant la macula, de préférence la dégénérescence maculaire liée à l'âge.

La présente invention concerne en outre une composition pharmaceutique comprenant un composé de l'invention et au moins un véhicule pharmaceutiquement acceptable.

Dans un mode de réalisation, la composition pharmaceutique de l'invention comprend un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci, de préférence le mirabégron, et au moins un véhicule pharmaceutiquement acceptable.

Dans un mode de réalisation, la composition pharmaceutique de l'invention est utilisée pour le traitement d'une maladie rétinienne affectant la macula, de préférence la dégénérescence maculaire liée à l'âge.

La présente invention concerne également un médicament comprenant un composé de l'invention.

Dans un mode de réalisation, le médicament de l'invention comprend un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci selon l'invention, de préférence le mirabégron, une composition ou une composition pharmaceutique de la présente invention.

Dans un mode de réalisation, le médicament de l'invention est utilisé pour le traitement d'une maladie rétinienne affectant la macula, de préférence la dégénérescence maculaire liée à l'âge.

De préférence, la composition, la composition pharmaceutique ou le médicament de la présente invention comprennent une quantité thérapeutiquement efficace d'un composé de l'invention, de préférence un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci, de préférence le mirabégron.

Dans un mode de réalisation, l'anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci de la présente invention, de préférence le mirabégron, est utilisé en combinaison avec au moins un autre agent thérapeutique pour traiter une maladie rétinienne affectant la macula, de préférence la dégénérescence maculaire liée à l'âge.

Des exemples d'autres agents thérapeutiques pour traiter la dégénérescence maculaire liée à l'âge incluent, mais ne sont pas limités à, des agents anti-vasoprolifératifs tels que le ranibizumab (lucentis) ou le bévacizumab (avastin), des agents anti-angiogéniques tels que le VEGF trap (regeneron), le bévasiranib ou des inhibiteurs des tyrosines kinases.

Dans un mode de réalisation, la quantité thérapeutiquement efficace va d'environ 1 à 10000 mg/mL de composition, composition pharmaceutique ou médicament de l'invention, de préférence d'environ 5 à environ 5000 mg/mL, de préférence d'environ 10 à environ 2000 mg/mL, de préférence d'environ 20 à environ 100 mg/mL de composition, composition pharmaceutique ou médicament de l'invention.

Dans un mode de réalisation, la quantité thérapeutiquement efficace va d'environ 1 à 10000 mg/g de composition, composition pharmaceutique ou médicament de l'invention, de préférence d'environ 5 à environ 5000 mg/g, de préférence d'environ 10 à environ 2000 mg/g, de préférence d'environ 20 à environ 100 mg/g de composition, composition pharmaceutique ou médicament de l'invention.

Il est entendu que l'usage journalier total de l'anilide de l'acide (R)-2-(2-aminothiazol-4-yl)-4'-[2-[(2-hydroxy-2-phényléthyl)amino]éthyl]acétique ou l'analogue ou le sel ou le solvate pharmaceutiquement acceptable de celui-ci, de préférence le mirabégron, de la composition, de la composition pharmaceutique ou du médicament de l'invention sera ajusté par le médecin traitant dans le cadre de son avis médical. La dose thérapeutiquement efficace spécifique à chaque patient dépendra d'une variété de facteurs incluant le trouble traité et sa sévérité ; l'activité du composé utilisé ; la composition spécifique utilisée ; l'âge, le poids, l'état de santé général, le sexe et le régime alimentaire du patient, la durée et le mode d'administration ; la durée du traitement ; les médicaments utilisés en combinaison ou coïncidents avec le composé utilisé, et d'autres facteurs similaires connus dans le domaine médical. Par exemple, il est courant dans ce domaine de commencer avec des doses de composés inférieures aux doses recommandées pour atteindre l'effet thérapeutique désiré et de graduellement augmenter le dosage jusqu'à ce que l'effet soit atteint. Cependant, le dosage journalier des composés peut varier sur une vaste plage allant d'environ 1 à environ 10000 mg par adulte et par jour, de préférence d'environ 5 à environ 5000, de préférence d'environ 10 à environ 2000 mg, plus préférentiellement d'environ 20 à environ 100 mg par adulte et par jour. De préférence, la composition comprend 1, 10, 20, 50, 100, 250, 500, 1000 et 2000 mg de l'ingrédient actif pour l'ajustement symptomatique du dosage à administrer au patient à traiter. Un médicament contient typiquement d'environ 1 à environ 10000 mg d'ingrédient actif, de préférence de 5 à 5000, de préférence de 10 à 2000 mg d'ingrédient actif. Une quantité efficace du médicament est ordinairement fournie à une dose allant d'environ 0.01 mg/kg à environ 100 mg/kg de poids corporel par jour, de préférence d'environ 0.05 mg/kg à environ 40 mg/kg, de préférence d'environ 0.1 mg/kg à 20 mg/kg de poids corporel par jour, plus préférentiellement d'environ 0.2 à environ 1 mg/kg de poids corporel par jour.

Dans un mode de réalisation, la dose journalière du composé de l'invention, de préférence le mirabégron, de la composition, de la composition pharmaceutique ou du médicament de la présente invention est ajustée en fonction des potentiels troubles rénaux et/ou hépatiques du sujet.

Dans un mode de réalisation, la dose journalière totale de l'anilide de l'acide (R)-2-(2-aminothiazol-4-yl)-4'-[2-[(2-hydroxy-2-phényléthyl)amino]éthyl]acétique ou l'analogue ou le sel ou le solvate pharmaceutiquement acceptable de celui-ci, de préférence le mirabégron, va d'environ 1 mg à environ 100 mg, de préférence d'environ 10 mg à environ 80 mg, de préférence d'environ 20 mg à environ 60 mg.

Dans un mode particulier de réalisation, la dose journalière totale initiale de l'anilide de l'acide (R)-2-(2-aminothiazol-4-yl)-4'-[2-[(2-hydroxy-2-phényléthyl)amino]éthyl]acétique ou l'analogue ou le sel ou le solvate pharmaceutiquement acceptable de celui-ci, de préférence le mirabégron, va d'environ 10 mg à environ 50 mg, de préférence d'environ 20 mg à environ 30 mg, de préférence est d'environ 25 mg. Dans un autre mode particulier de réalisation, la dose journalière totale de maintenance de l'anilide de l'acide (R)-2-(2-aminothiazol-4-yl)-4'-[2-[(2-hydroxy-2-phényléthyl)amino]éthyl]acétique ou l'analogue ou le sel ou le solvate pharmaceutiquement acceptable de celui-ci, de préférence le mirabégron, va d'environ 20 mg à environ 80 mg, de préférence d'environ 40 mg à environ 60 mg, de préférence environ 50 mg.

Dans un mode particulier de réalisation, l'anilide de l'acide (R)-2-(2-aminothiazol-4-yl)-4'-[2-[(2-hydroxy-2-phényléthyl)amino]éthyl]acétique ou l'analogue ou le sel ou le solvate pharmaceutiquement acceptable de celui-ci, de préférence le mirabégron, la composition, la composition pharmaceutique ou le médicament de l'invention est administré à une dose d'environ 25 mg. Dans un autre mode particulier de réalisation, l'anilide de l'acide (R)-2-(2-aminothiazol-4-yl)-4'-[2-[(2-hydroxy-2-phényléthyl)amino]éthyl]acétique ou l'analogue ou le sel ou le solvate pharmaceutiquement acceptable de celui-ci, de préférence le mirabégron, la composition, la composition pharmaceutique ou le médicament de l'invention est administré à une dose d'environ 50 mg.

Dans un mode de réalisation, le médicament de l'invention contient environ 25 mg du composé, de la composition ou de la composition pharmaceutique de l'invention. Dans un autre mode de réalisation, le médicament de l'invention contient environ 50 mg du composé, de la composition ou de la composition pharmaceutique de l'invention.

Dans un mode de réalisation, le composé de l'invention, de préférence un anilide de l'acide (R)-2-(2-aminothiazol-4-yl)-4'-[2-[(2-hydroxy-2-phényléthyl)amino]éthyl]acétique ou l'analogue ou le sel ou solvate pharmaceutiquement acceptable de celui-ci, de préférence le mirabégron, la composition, la composition pharmaceutique ou le médicament de la présente invention, seul ou en combinaison avec un autre agent thérapeutique, peut être administré sous forme de dosage unitaire, en mélange avec les supports pharmaceutiques classiques, aux animaux et aux êtres humains. Les formes d'administration unitaire appropriées comprennent les formes d'administration orales telles que les comprimés, gélules, poudres, granulés et suspensions ou solutions orales, les formes d'administration sublinguales ou buccales, les aérosols, les implants, les formes d'administration sous cutanées, transdermiques, topiques, intrapéritonéales, intraveineuses, intrathécales, intraoculaires et intranasales, et les formes d'administration rectales.

Dans un mode de réalisation, la composition, la composition pharmaceutique ou le médicament de la présente invention comprend un ou des véhicules pharmaceutiquement acceptables pour une formulation adaptée à une administration orale.

Des exemples de formes adaptées à une administration orale incluent, mais ne sont pas limités aux comprimés (dont les comprimés à libération prolongée), gélules, poudres, granulés, pilules (dont les pilules enrobées de sucre), capsules (dont les capsules de gélatine souple), suspensions orales, solutions buvables, et autres formes similaires.

Dans un mode de réalisation, la composition, la composition pharmaceutique ou le médicament de la présente invention comprend un ou des véhicules pharmaceutiquement acceptables pour une formulation adaptée à une administration topique. Dans un mode particulier de réalisation, la composition, la composition pharmaceutique ou le médicament de la présente invention comprend un ou des véhicules pharmaceutiquement acceptables pour une formulation adaptée à une administration topique dans l'œil.

Des exemples de formes adaptées à une administration topique incluent, mais ne sont pas limités aux compositions sous forme liquide, pâteuse, ou solide et, plus particulièrement, sous forme de solutions aqueuses, de collyres, de gouttes, de dispersions, de sprays, ou de microcapsules, micro- ou nanoparticules ou de patchs polymériques ou gélifiés permettant une libération contrôlée.

Dans un mode de réalisation, la composition, la composition pharmaceutique ou le médicament de la présente invention comprend un ou des véhicules pharmaceutiquement acceptables pour une formulation susceptible d'être injectée. Dans un mode particulier de réalisation, la composition, la composition pharmaceutique ou le médicament de la présente invention a une forme adaptée pour une injection intraoculaire, de préférence pour une injection intravitrée.

Des exemples de formes adaptées à une administration par injection incluent, mais ne sont pas limités aux solutions aqueuses stériles, dispersions, émulsions, suspensions, formes solides appropriées pour la préparation de solutions ou suspensions par addition d'un liquide avant utilisation telles que, par exemple, des poudres.

Dans un mode de réalisation, le composé de l'invention, de préférence un anilide de l'acide (R)-2-(2-aminothiazol-4-yl)-4'-[2-[(2-hydroxy-2-phényléthyl)amino]éthyl]acétique ou l'analogue ou le sel ou solvate pharmaceutiquement acceptable de celui-ci, de préférence le mirabégron, la composition, la composition pharmaceutique ou le médicament de la présente invention est administré au sujet au moins une fois par jour. Par exemple, le composé, la composition, la composition pharmaceutique ou le médicament de l'invention peut être administré une fois par jour, deux fois ou trois fois par jour. De préférence, le composé, la composition, la composition pharmaceutique ou le médicament de l'invention est administré une fois par jour.

Dans un autre mode de réalisation, le composé de l'invention, de préférence un anilide de l'acide (R)-2-(2-aminothiazol-4-yl)-4'-[2-[(2-hydroxy-2-phényléthyl)amino]éthyl]acétique ou l'analogue ou le sel ou solvate pharmaceutiquement acceptable de celui-ci, de préférence le mirabégron, la composition, la composition pharmaceutique ou le médicament de la présente invention est administré au sujet au moins une fois par semaine. Par exemple, le composé, la composition, la composition pharmaceutique ou le médicament de l'invention peut être administré une fois par semaine, deux fois, trois fois, quatre fois ou jusqu'à sept fois par semaine.

Dans un autre mode de réalisation, le composé de l'invention, de préférence un anilide de l'acide (R)-2-(2-aminothiazol-4-yl)-4'-[2-[(2-hydroxy-2-phényléthyl)amino]éthyl]acétique ou l'analogue ou le sel ou solvate pharmaceutiquement acceptable de celui-ci, de préférence le mirabégron, la composition, la composition pharmaceutique ou le médicament de la présente invention est administré au sujet au plus une fois par mois. Par exemple, le composé, la composition, la composition pharmaceutique ou le médicament de l'invention peut être administré une fois par mois, une fois tous les deux mois, une fois par trimestre, deux fois par an ou une fois par an.

La présente demande concerne également une méthode pour traiter une maladie rétinienne, de préférence la dégénérescence maculaire liée à l'âge, chez un sujet qui en a besoin comprenant l'administration au sujet d'une quantité thérapeutiquement efficace d'un composé de l'invention comme décrit ci-dessus, de préférence un anilide de l'acide (R)-2-(2-aminothiazol-4-yl)-4'-[2-[(2-hydroxy-2-phényléthyl)amino]éthyl]acétique ou un analogue ou un sel ou solvate pharmaceutiquement acceptable de celui-ci, de préférence le mirabégron.

Dans un mode de réalisation, la composition, composition pharmaceutique ou médicament de l'invention est administré au sujet.

La présente demande concerne également une méthode pour diminuer le pH lysosomal dans les cellules de l'épithélium pigmentaire rétinien comprenant l'administration d'une composition comprenant un composé de l'invention comme décrit ci-dessus, de préférence un anilide de l'acide (R)-2-(2-aminothiazol-4-yl)-4'-[2-[(2-hydroxy-2-phényléthyl)amino]éthyl]acétique ou un analogue ou un sel ou solvate pharmaceutiquement acceptable de celui-ci, de préférence le mirabégron.

La présente demande concerne en outre une méthode pour augmenter la digestion des segments externes des photorécepteurs de l'épithélium pigmentaire rétinien comprenant l'administration d'une composition comprenant un composé de l'invention comme décrit ci-dessus, de préférence un anilide de l'acide (R)-2-(2-aminothiazol-4-yl)-4'-[2-[(2-hydroxy-2-phényléthyl)amino]éthyl]acétique ou un analogue ou un sel ou solvate pharmaceutiquement acceptable de celui-ci, de préférence le mirabégron.

La présente demande concerne également une méthode pour diminuer l'accumulation de lipofuscine dans les cellules de l'épithélium pigmentaire rétinien comprenant l'administration d'une composition comprenant un composé de l'invention comme décrit ci-dessus, de préférence un anilide de l'acide (R)-2-(2-aminothiazol-4-yl)-4'-[2-[(2-hydroxy-2-phényléthyl)amino]éthyl]acétique ou un analogue ou un sel ou solvate pharmaceutiquement acceptable de celui-ci, de préférence le mirabégron.

La présente invention concerne également un kit comprenant un anilide de l'acide (R)-2-(2-aminothiazol-4-yl)-4'-[2-[(2-hydroxy-2-phényléthyl)amino]éthyl]acétique ou un analogue ou un sel ou solvate pharmaceutiquement acceptable de celui-ci, une composition, une composition pharmaceutique ou un médicament comme décrit ci-dessus.

Selon un mode de réalisation, le kit comprend également un appareil servant à administrer le composé, la composition, la composition pharmaceutique ou le médicament à un sujet.

Selon un mode de réalisation, le kit comprend en plus les instructions pour l'administration du composé, de la composition, de la composition pharmaceutique ou du médicament audit sujet.

Dans un mode de réalisation, le kit comprend un agent thérapeutique additionnel. Selon un mode de réalisation, l'agent thérapeutique additionnel est un autre agent pour le traitement de la maladie rétinienne selon l'invention.

Dans un mode de réalisation, l'agent thérapeutique additionnel a une forme adaptée à la même voie d'administration que le composé, la composition, la composition pharmaceutique ou le médicament de l'invention. Dans un autre mode de réalisation, l'agent thérapeutique additionnel a une forme adaptée à une voie d'administration différente de celle du composé, de la composition, de la composition pharmaceutique ou du médicament de l'invention.

### BRÈVE DESCRIPTION DES FIGURES

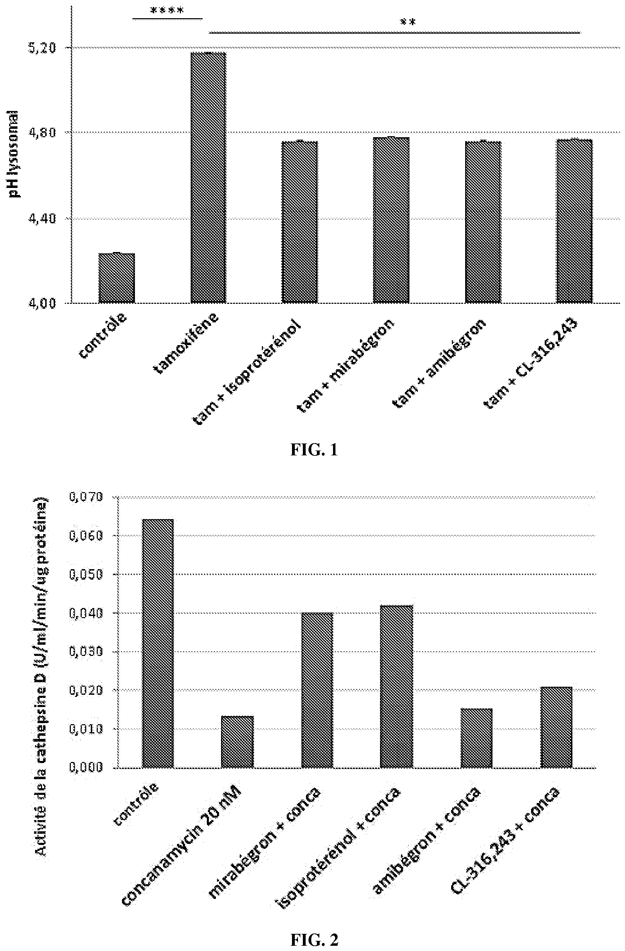

La **Figure 1** est un histogramme montrant l'effet des agonistes aux récepteurs adrénergiques isoproterenol, mirabegron, amibegron et CL-316,243 sur le pH lysosomal de cellules traitées par du tamoxifen. Les résultats ont été comparés statistiquement par une Anova et un test de Dunnett's. *****p*<0.0001, ***p*<0.01.
La **Figure 2** est un histogramme montrant l'effet des agonistes aux récepteurs adrénergiques mirabegron, isoproterenol, amibegron et CL-316,243 sur l'activité de la cathepsine D de cellules traitées par de la concamycine.

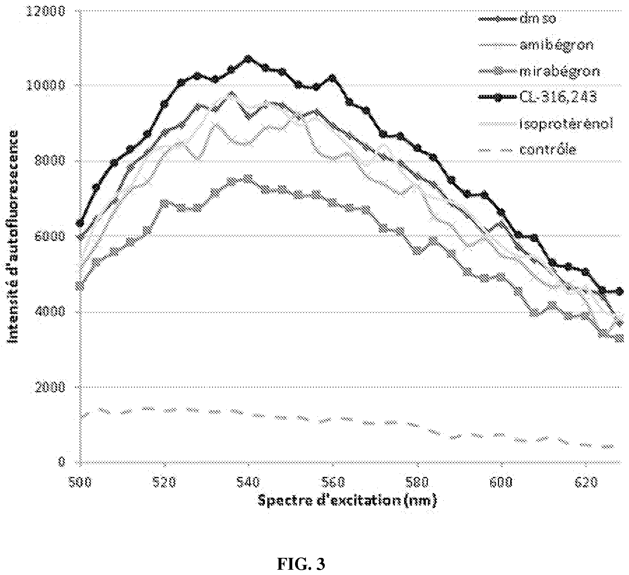

La **Figure 3** est un graphique montrant l'intensité d'autofluorescence de la lipofuscine après deux semaines de co-traitement des cellules de l'EPR par des segments externes oxydés et par les agonistes aux récepteurs adrénergiques amibegron, mirabegron, CL-316,243 et isoproterenol. Des cellules co-traitées par des segments externes oxydés et par du DMSO servent de contrôle.

### EXEMPLES

La présente invention se comprendra mieux à la lecture des exemples suivants qui illustrent non-limitativement l'invention.

### Exemple 1

### Matériel et Méthodes

### Culture primaire d'EPR de porc

Les yeux de porc sont livrés à l'Institut de la Vision dans un milieu froid en provenance d'un abattoir local. Les yeux sont disséqués de manière à enlever le segment antérieur de l'œil, le vitrée et la rétine neurale. Les globes oculaires sont ensuite lavés deux fois au PBS, remplis de trypsine (0,25% en PBS) et incubés à 37°C pendant 1h15. Les cellules de l'EPR sont alors récupérées par pipetages répétés, centrifugées afin d'éliminer la trypsine, et resuspendues dans du milieu de culture DMEM additionné de 20% de sérum de veau fétal (DMEM20%SVF). Les cellules de chaque œil sont alors ensemencées dans une boîte de Pétri de 6 cm de diamètre, cultivées en atmosphère contenant 5% de CO2 à 37°C, et le milieu de culture est changé après 24 heures et 4 jours in vitro. Après une semaine, les cellules atteignent la confluence et peuvent alors être passées.

### Alcalinisation et mesure du pH lysosomal (pH_{L}) de l'EPR

Après une semaine en culture, les cellules sont traitées à la trypsine et transférées dans une plaque 96 puits à fond noir à une densité cellulaire de 1,5 x 10⁵ cellules/cm² dans du DMEM2%SVF. Après 24 heures, les cellules sont traitées par un agoniste béta-adrénergique (mirabégron, amibégron, clenbutérol ou isoprotérénol à 1 pM ou CL-316,243 à 20 nM), et 5 minutes plus tard par le tamoxifène (15 µM), et le pH_{L} est mesuré après 20 autres minutes. Cette mesure est réalisée en utilisant un indicateur coloré (Lysosensor Yellow/BlueDND-160) présentant une excitabilité à 329 et 384 nm et permettant une mesure des variations de pH dans les organelles acides indépendante de la concentration du colorant. Pour réaliser la mesure du pH_{L}, les cellules sont incubées par le colorant pendant 5 minutes à 37°C, et la fluorescence émise par le colorant est mesurée sur un lecteur de plaque. Le ratio de lumière excitée à 329/384 nm est alors converti en pH en utilisant une gamme de calibration (pH 4 à pH 6) faite dans un tampon KCl en présence de 10 µM de monensine et 20 µM de nigéricine, deux ionophores.

### Mesure de l'activité de la cathepsine D

Après une semaine en culture, les cellules sont traitées à la trypsine et transférées dans des boîtes de Pétri de 3,5 cm de diamètre à une densité cellulaire de 1,5 x 10⁵ cellules/cm² dans du DMEM2%SVF. Après 24 heures, les cellules sont traitées par 20 nM de concamycine afin d'inhiber l'activité de la cathepsine D, ainsi que par un agoniste béta-adrénergique. Après 24 heures de traitement, les cellules sont lavées par du PBS puis transférées dans un tampon d'extraction sur glace. L'extrait cellulaire est centrifugé à 2000 tours/min à 4°C pendant 10 minutes et le surnageant correspondant à la partie cytosolique est congelé à -80°C jusqu'à la mesure de l'activité enzymatique. L'activité de la cathepsine D est mesurée par la méthode de Anson (J Gen Physiol. 1938, 22(1): 79-89) que nous avons adaptée à notre schéma expérimental. En résumé, l'extrait cytosolique est incubé pendant 10 minutes à 37°C dans une solution d'hémoglobine (2,5% dans 400 mM de tampon citrate à pH 2.8). La réaction est stoppée par ajout de 5% d'acide trichloracétique et le mélange est centrifugé. La densité optique du surnageant contenant les produits de dégradation de l'hémoglobine est mesurée à 280 nm. L'absorbance est corrigée en soustrayant celle du témoin, préparé comme précédemment mais en ajoutant l'hémoglobine après avec arrêté la réaction enzymatique. Une unité de cathepsine D est alors définie comme étant la quantité d'enzyme nécessaire pour induire un changement d'absorbance de 1 à 280 nm pour 60 minutes d'incubation en utilisant les conditions expérimentales décrites ci-dessus. La concentration protéique des lysats cellulaires est mesurée selon la méthode de Bradford afin de normaliser les résultats.

### Préparation de segments externes de photorécepteurs (SEP) de porc

Des rétines de porc sont prélevées en chambre noire sous lumière rouge. Les SEP sont séparés des rétines sur gradient de sucrose comme décrit ci-après. En résumé, les rétines de porc sont homogénéisées dans une solution contenant 20% de sucrose, 20 mM de Tris-acétate à pH 7.2, 2 mM de MgCl2, 10 mM de glucose and 5 mM de taurine. Les échantillons sont alors déposés sur un gradient continu de sucrose (25 à 60%) contenant 20 mM de Tris acétate à pH 7.2, 10 mM de glucose et 5 mM de taurine, et centrifugé à 25000 tours/min à 4°C pendant 2h. Les bandes roses obtenues correspondent aux SEP et sont alors prélevées puis congelées à -80°C jusqu'à utilisation.

Afin d'obtenir des SEP oxydés (SEP-ox), les SEP sont exposés aux ultra-violets (λ=312 nm) pendant 3h. Ils sont ensuite lavés dans du PBS, centrifugés à 5000 tours/min et resuspendus dans du DMEM20%FCS contenant 2.5% de sucrose.

### Modèle in vitro d'accumulation de lipofuscine dans l'EPR

Après une semaine en culture, les cellules sont traitées à la trypsine et transférées dans une plaque 96 puits à fond clair à une densité cellulaire de 1,5 x 10⁵ cellules/cm² dans du DMEM2%SVF.

Afin d'induire une accumulation de matériel de type lipofuscine dans les cellules d'EPR, ces dernières sont traitées 3 fois par semaine par 5x10⁶ SEP-ox dans du DMEM20%FCS contenant 2.5% de sucrose pendant deux semaines. Parallèlement les cellules sont traitées ou non par un antagoniste béta-adrénergique. L'autofluorescence induite par l'accumulation de lipofuscine est mesurée par un lecteur de plaque (excitation à 480 nm et émission entre 500 et 700 nm correspondant au spectre d'émission de la lipofuscine).

### Résultats

### Effet des agonistes sur le pH lysosomal

Les molécules testées dans les expériences consistent en trois agonistes spécifiques du récepteur adrénergique béta-3 (Mirabégron, Amibégron, CL-316,243), et un agoniste non spécifique (isoprotérénol).

Ces molécules ont été testées dans un modèle cellulaire d'alcalinisation du pH lysosomal de l'EPR induite par traitement des cellules par 15 µM de tamoxifen et provoquant une hausse du pH de l'ordre de 1 unité de pH en 20 minutes.

Toutes les molécules testées ont permis d'obtenir un effet maximum de près de 50% sur la ré-acidification du pH à 1 pM à l'exception du CL-316,243 efficace à partir de 20 nM (Figure 1).

### Effet des agonistes sur l'activité de la cathepsine D

La cathepsine D est l'enzyme protéolytique lysosomale majoritairement présente dans l'épithélium pigmentaire rétinien participant à la digestion des segments externes des photorécepteurs. Son activité dépend de la protonation de l'acide aminé acide aspartique de son site actif et de sa conformation, qui requièrent tous deux un environnement acide. Ainsi, l'étude de l'effet des molécules testées sur l'activité de la cathepsine D permet de déterminer l'effet de ces molécules sur l'activité des enzymes lysosomales.

Excepté l'amibégron, le traitement des cellules de l'EPR avec toutes les molécules testées permet la restauration partielle de l'activité de la cathepsine D (Figure 2). En particulier, le traitement avec le mirabégron multiplie par 3 l'activité de l'enzyme (0,040 unité/ml d'enzyme/min/µg de protéines) comparé au témoin négatif que constitue le traitement par la concanamycine (0,013 unité/ml d'enzyme/min/µg de protéines).

### Effet des agonistes sur l'accumulation de lipofuscine dans l'EPR

Afin de vérifier si les différents agonistes béta-adrénergiques sont capables d'agir également sur l'accumulation de lipofuscine, nous les avons testés dans un modèle cellulaire dans lequel cette accumulation est induite par traitement des cellules d'EPR par des SE oxydés par exposition aux U.V. tous les deux jours. Les agonistes ont été ajoutés à 10 µM au milieu de culture au moment des traitements par les SE. L'accumulation de lipofuscine a été mesurée après deux semaines de co-traitement par mesure de l'autofluorescence des cellules.

Les résultats montrent qu'en deux semaines seul le mirabégron est capable de réduire de près de 20% l'accumulation de lipofuscine par les cellules d'EPR (Figure 3).

## Revendications

1. Anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation dans le traitement d'une maladie rétinienne chez un sujet, ledit anilide de l'acide (R)-2-(2-aminothiazol-4-yl)-4'-[2-[(2-hydroxy-2-phényléthyl)amino]éthyl]acétique étant le mirabégron, un sel ou solvate pharmaceutiquement acceptable de celui-ci, et dans lequel ladite maladie rétinienne est une maladie affectant la macula.

2. Anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation selon la revendication 1, dans lequel ladite maladie rétinienne est la dégénérescence maculaire liée à l'âge, de préférence la dégénérescence maculaire liée à l'âge de type atrophique.

3. Composition pharmaceutique comprenant un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation selon l'une quelconque des revendications **1** à **2** et au moins un véhicule pharmaceutiquement acceptable.

4. Médicament comprenant un anilide de l'acide (R)-2-(2-aminothiazol-4-yl)-4'-[2-[(2-hydroxy-2-phényléthyl)amino]éthyl]acétique ou un sel ou solvate pharmaceutiquement acceptable de celui-ci pour son utilisation selon l'une quelconque des revendications **1** à **2.**

5. Composition pharmaceutique ou médicament pour son utilisation selon l'une quelconque des revendications **3** ou **4, caractérisé en ce qu'**il est destiné à être administré au sujet qui en a besoin par voie orale ou par voie topique.

6. Kit comprenant un composé pour son utilisation selon l'une quelconque des revendications **1** à **2,** une composition pharmaceutique pour son utilisation selon l'une quelconque des revendications **3** et **5,** ou un médicament pour son utilisation selon l'une quelconque des revendications **4** et **5.**

7. Kit pour son utilisation selon la revendication **6, caractérisé en ce qu'**il comprend en plus un appareil servant à administrer le composé, la composition pharmaceutique ou le médicament à un sujet qui en a besoin, et optionnellement les instructions pour l'administration du composé, de la composition pharmaceutique ou du médicament audit sujet.

8. Mirabégron pour son utilisation pour le traitement de la DMLA.

## Patentansprüche

1. Anilid der (R)-2-(2-aminothiazol-4-yl)-4'-[2-[(2-hydroxy-2-phenylethyl)amino]ethyl]essigsäure oder ein pharmazeutisch akzeptables Salz oder Solvat desselben für seine Verwendung bei der Behandlung einer Retinaerkrankung einer Person, wobei das Anilid der (R)-2-(2-aminothiazol-4-yl)-4'-[2-[(2-hydroxy-2-phenylethyl)amino]ethyl]essigsäure das Mirabegron, ein pharmazeutisch akzeptables Salz oder Solvat desselben ist, und wobei die Retinaerkrankung eine Erkrankung ist, die die Makula betrifft.

2. Anilid der (R)-2-(2-aminothiazol-4-yl)-4'-[2-[(2-hydroxy-2-phenylethyl)amino]ethyl]essigsäure oder ein pharmazeutisch akzeptables Salz oder Solvat desselben für seine Verwendung nach Anspruch **1,** wobei die Retinaerkrankung die altersbedingte Makuladegeneration ist, vorzugsweise die altersbedingte Makuladegeneration vom atrophischen Typ.

3. Pharmazeutische Zusammensetzung, umfassend ein Anilid der (R)-2-(2-aminothiazol-4-yl)-4'-[2-[(2-hydroxy-2-phenylethyl)amino]ethyl]essigsäure oder ein pharmazeutisch akzeptables Salz oder Solvat desselben für seine Verwendung nach einem der Ansprüche **1** bis **2** und mindestens ein pharmazeutisch akzeptables Vehikel.

4. Arzneimittel, umfassend ein Anilid der (R)-2-(2-aminothiazol-4-yl)-4'-[2-[(2-hydroxy-2-phenylethyl)amino]ethyl]essigsäure oder ein pharmazeutisch akzeptables Salz oder Solvat desselben für seine Verwendung nach einem der Ansprüche **1** bis **2.**

5. Pharmazeutische Zusammensetzung oder Arzneimittel für seine Verwendung nach einem der Ansprüche **3** oder **4, dadurch gekennzeichnet, dass** es bestimmt ist, der Person, die es benötigt, auf oralem oder topischem Weg verabreicht zu werden.

6. Kit, umfassend eine Verbindung für ihre Verwendung nach einem der Ansprüche **1** bis **2,** eine pharmazeutische Zusammensetzung für ihre Verwendung nach einem der Ansprüche **3** und **5** oder ein Arzneimittel für seine Verwendung nach einem der Ansprüche **4** und **5.**

7. Kit für seine Verwendung nach Anspruch **6, dadurch gekennzeichnet, dass** es zusätzlich einen Apparat umfasst, der zur Verabreichung der Verbindung, der pharmazeutischen Zusammensetzung oder des Arzneimittels an eine Person dient, die dies benötigt, und optional die Anweisung für die Verabreichung der Verbindung, der pharmazeutischen Zusammensetzung oder des Arzneimittels an die Person.

8. Mirabegron für seine Verwendung zur Behandlung der AMD

## Claims

1. (R)-2-(2-aminothiazol-4-yl)-4'-[2-[(2-hydroxy-2-phenylethyl)amino]ethyl]acetic acid anilide or a pharmaceutically acceptable salt or solvate thereof for use in the treatment of a retinal disease in a subject, said (R)-2-(2-aminothiazol-4-yl)-4'-[2-[(2-hydroxy-2-phenylethyl)amino]ethyl]acetic acid anilide being mirabegron, a pharmaceutically acceptable salt or solvate thereof, and wherein said retinal disease is a disease affecting the macula.

2. (R)-2-(2-aminothiazol-4-yl)-4'-[2-[(2-hydroxy-2-phenylethyl)amino]ethyl]acetic acid anilide or a pharmaceutically acceptable salt or solvate thereof for use according to claim **1,** wherein said retinal disease is age-related macular degeneration, preferably atrophic age-related macular degeneration.

3. Pharmaceutical composition comprising an (R)-2-(2-aminothiazol-4-yl)-4'-[2-[(2-hydroxy-2-phenylethyl)amino]ethyl]acetic acid anilide or a pharmaceutically acceptable salt or solvate thereof for use according to any one of claims **1** to **2** and at least one pharmaceutically acceptable vehicle.

4. Medicament comprising an (R)-2-(2-aminothiazol-4-yl)-4'-[2-[(2-hydroxy-2-phenylethyl)amino]ethyl]acetic acid anilide or a pharmaceutically acceptable salt or solvate thereof for use according to any one of claims **1** to **2.**

5. Pharmaceutical composition or medicament for use according to any one of claims **3** or **4, characterized in that** it is intended to be administered to the subject in need thereof orally or topically.

6. Kit comprising a compound for use according to any one of claims **1** to **2,** a pharmaceutical composition for use according to any one of claims **3** and **5,** or a medicament for use according to any one of claims **4** and **5.**

7. Kit for use according to claim **6, characterized in that** it further comprises an apparatus for administering the compound, pharmaceutical composition or medicament to a subject in need thereof, and optionally the instructions for administering the compound, pharmaceutical composition or medicament to said subj ect.

8. Mirabegron for use for the treatment of AMD.
